# EUROPÄISCHE PATENTSCHRIFT

(11) **EP 3 437 581 B1**
(45) Veröffentlichungstag und Bekanntmachung des Hinweises auf die Patenterteilung: **30.11.2022**
(21) Anmeldenummer: 18187189.8
(22) Anmeldetag: 03.08.2018
(51) Int. Cl.: A61B 18/14

(54) **ELEKTRODENEINHEIT FÜR EIN MEDIZINISCHES RESEKTOSKOP**
ELECTRODE UNIT FOR A MEDICAL RESECTOSCOPE
UNITÉ D'ÉLECTRODE POUR UN RÉSECTOSCOPE MÉDICAL

(30) Priorität: 04.08.2017 DE 102017117749
(43) Veröffentlichungstag der Anmeldung: 06.02.2019
(73) Patentinhaber: Olympus Winter & Ibe GmbH, 22045 Hamburg (DE)
(72) Erfinder: BROCKMANN, Christian, 21279 Hollenstedt (DE); Miki, Jun, Tokyo, Meguro-ku 153-0051 (JP)
(74) Vertreter: Hoener, Matthias

(56) Entgegenhaltungen:
- EP-A1- 0 481 310
- EP-A1- 1 974 683
- Anonymous: "YOUR CHOICE FOR BPH Resection, Vaporization, Enucleation - Individual PLASMA Treatment", , 1. März 2017 (2017-03-01), XP055513421, Gefunden im Internet: URL:https://www.olympus-europa.com/medical /rmt/media/en/Content/Content-MSD/Document s/Brochures/PLASMA_Brochure_EN_16319.pdf [gefunden am 2018-10-08]
- Anonymous: "Surgical Tissue Management System Electrosurgical Generator", , 1. Januar 2014 (2014-01-01), XP055513415, Gefunden im Internet: URL:https://www.olympus.pl/medical/rmt/med ia/en/Content/Content-MSD/Documents/Brochu res/ESG-400_Brochure_EN_20140306.pdf [gefunden am 2018-10-08]

## Beschreibung

Die Erfindung betrifft eine Elektrodeneinheit für ein medizinisches Resektoskop gemäß dem Oberbegriff des Anspruchs 1.

Gattungsgemäße Elektrodeneinheiten werden in der Chirurgie zum Entfernen von Gewebe aus dem Körperinneren eines Patienten eingesetzt. Insbesondere werden solche mit einer mit Hochfrequenzstrom beaufschlagbaren Schneidelektrode ausgestatteten Elektrodeneinheiten für urologische Eingriffe verwendet, bei denen aus dem Bereich der Harnblase oder der Harnröhre krankhaftes oder wucherndes Gewebe entfernt wird. Für solche Eingriffe wird die Elektrodeneinheit an ein Resektoskop angeschlossen, dessen Schaft durch den Harnleiter des Patienten bis in das Operationsgebiet vorgeschoben wird. Im Operationsgebiet kann die distal an einem langgestreckten Elektrodenschaft getragene Schneidelektrode durch Betätigung eines proximal an dem Resektoskop angeordneten Handgriffs in distale oder proximale Richtung schiebend oder ziehend bewegt werden. Bei Beaufschlagung der Schneidelektrode mit einem hochfrequenten Wechselstrom kann das zu entfernende Gewebe, wie zum Beispiel ein Tumor oder Prostatagewebe, durch lokale Hitzeentwicklung von der Blasenwand oder von der Harnröhre abgeschnitten oder abgetragen werden.

Gattungsgemäße Elektrodeneinheiten weisen einen geraden langgestreckten Elektrodenschaft auf, der an seinem distalen Ende einen sich meist gabelförmig in zwei Tragarmen aufteilenden ausgebildeten Elektrodenträger aufweist, an dem eine mit Hochfrequenzstrom beaufschlagbare Schneidelektrode angeordnet ist. Die Schneidelektrode kann - je nach Präferenz des Operateurs oder Einsatzzweck des Instrumentes - unterschiedlich geformt sein, zum Beispiel schlingenförmig oder nadelförmig.

Für die transurethrale Resektion der Prostata ist die Schneidelektrode typischerweise als Schneidschlinge ausgeführt, die sich in einem rechten Winkel zur Schaftachse des Elektrodenschaftes zwischen den zwei Tragarmen des Elektrodenträgers erstreckt. Bei einer Vor- und Zurückbewegung der Elektrodeneinheit in dem Außenschaft des Resektoskopes wird die Schneidelektrode distal vor die Öffnung des Außenrohres geschoben bzw. dicht an die distale Öffnung des Außenschaftes herangezogen. Dabei wird mit der Schlinge Prostatagewebe ergriffen und durch Beaufschlagung der Schlinge mit Hochfrequenzstrom so stark erhitzt, dass sich die Elektrode durch das Gewebe schneidet.

Elektrodeneinheiten der gattungsgemäßen Art werden zudem auch für die Entfernung von Tumoren oder anderen krankhaften Gewebebereichen aus der Harnblase verwendet. Dafür kommen bevorzugt nadelförmige bzw. stabförmige Schneidelektroden zum Einsatz. Zur Vorbereitung der Entfernung von Gewebe aus der Harnblase wird die Harnblasenwand üblicherweise zunächst mit der Schneidelektrode rund um das zystenartig an der Blasenwand haftende Gewebe einbrennend kontaktiert, um den zu entfernenden Gewebebereich zu markieren. Anschließend wird der zu entfernende Gewebeblock innerhalb des markierten Bereiches dicht an der Blasenwand geschnitten, und möglichst in einem zusammenhängenden Stück von der Blasenwand abgetrennt. Dabei wird die Schneidelektrode mit Kraft unter den Gewebeblock gedrückt, um den Schnittbereich vorzuspannen, was die Abtrennung des Gewebes von der Blasenwand erleichtert.

Für diese En-Bloc-Resektion mit gattungsgemäßen Elektrodeneinheiten hat sich eine Operationstechnik etabliert, bei der das zu entfernende Gewebe mit den isolierten Seitenkanten der Schneidelektrode zunächst unter leichter seitlicher Verkippung des Resektoskopes unter Spannung gebracht wird und dann mit einer Kombination aus Drehung und axialer Bewegung der Elektrodeneinheit an die Wirkfläche der Schneidelektrode geführt wird. Da die Schneidelektroden und die Verbindung der Schneidelektroden mit dem Elektrodenträger in ihrer herkömmlichen Ausführung nicht für hohe mechanische Belastungen ausgelegt sind, kann es beim kraftvollen Vorspannen des Gewebes zu einem erhöhtem Verschleiß der Schneidelektrode und ihrer Befestigungsstruktur an dem Elektrodenträger kommen.

EP 0 481 310 A1 offenbart eine Elektrodenanordnung für ein Resektoskop gemäß dem Stand der Technik.

Der vorliegenden Erfindung liegt daher die Aufgabe zu Grunde, eine verbesserte Elektrodeneinheit der eingangs beschriebenen Art bereitzustellen, die ohne Gefahr einer mechanischen Überlastung der Schneidelektrode eine kraftvolle Manipulation des zu entfernenden Gewebes erlaubt.

Diese Aufgabe wird gelöst durch eine Elektrodeneinheit mit den Merkmalen des Anspruchs 1. Vorteilhafte Ausgestaltungen sind in den Unteransprüchen angegeben.

Erfindungsgemäß ist eine Elektrodeneinheit für ein medizinisches Resektoskop zur elektrochirurgischen Resektion von Gewebe in einem mit Flüssigkeit gefüllten Hohlraum eines Patienten, aufweisend einen langgestreckten stabförmigen Elektrodenschaft, der an seinem distalen Ende einen Elektrodenträger und eine an dem Elektrodenträger befestigte, mit Wechselstrom beaufschlagbare Schneidelektrode aufweist, wobei an dem Elektrodenträger ein zur Abstützung von Gewebe ausgebildetes, die Schneidelektrode distal überragendes Spatelelement angeordnet ist, das zwei zu beiden Seiten der Schneidelektrode sich nach distal erstreckende, Außenkanten des Spatelelementes ausbildende Stützflanken aufweist, von denen mindestens eine zur Ausformung einer Taille im Bereich der Schneidelektrode eine nach innen geformte Einbuchtung ausbildet. Vorzugsweise ist vorgesehen, dass die zwei Stützflanken jeweils eine nach innen geformte Ausbuchtung ausbilden, um auf beiden Seiten der Schneidelektrode die im Folgenden näher erläuterte vorteilhafte Stützwirkung erzielen zu können.

Mit dem erfindungsgemäßen Spatelelement kann das zu zerschneidende Gewebe kraftvoll zur Seite gedrückt werden, sodass der mit der Schneidelektrode durchgeführte chirurgische Schnitt unter Vorspannung durchgeführt werden kann, ohne die Schneidelektrode dabei mechanisch zu überlasten. Mit der Taillenbildung des Spatelelementes kann das Gewebe mit den sich vor und hinter der Taille befindlichen, seitlich in größerem Abstand zur Schneidelektrode angeordneten Bereichen der seitlichen Stützflanken unter Spannung gehalten werden, während sich der zu schneidende Gewebebereich in die Taille legt und damit der Abstand des zu schneidenden Gewebes zur Schneidelektrode verringert wird.

Wie im Folgenden näher erläutert, ist vorzugsweise vorgesehen, dass die nach innen geformten Einbuchtungen der Stützflanken sich in einer Ebene mit den Stützflanken erstrecken.

Grundsätzlich ist denkbar, dass die die Außenkanten des Spatelelementes ausbildenden Stützflanken durch zwei zur Taillenausbildung nach innen gebogene Drahtstücke ausgebildet sind, die sich seitlich der Schneidelektrode nach distal erstrecken. Wie im Weiteren erläutert, können die Stützflanken jedoch auch durch die Kanten eines flächigen Spatelkörpers ausgebildet sein. Die besondere Eignung des Spatelelementes für die mechanische Vorspannung des zu entfernenden Gewebes ergibt sich durch die nach innen geformten Einbuchtungen der seitlichen Flanken.

Bei der Entfernung eines Tumors aus der Harnblase eines Patienten wird die Blasenwand rund um den Tumor üblicherweise zunächst durch flach in das Gewebe eintauchende Kontakte mit der mit Hochfrequenzstrom beaufschlagten Schneidelektrode markiert. Mit dem Spatelelement kann das Tumorgewebe dabei nach vorne abgestützt werden, um möglichst eng anliegende Markierungen in die Blasenwand einzubringen. Zur distalen Abstützung des zu entfernenden Gewebes ist in einer Ausführungsform daran gedacht, dass das Spatelelement an seinem distalen Ende eine gerade, sich quer zur Schaftachse erstreckende Stirnflanke aufweist. Alternativ kann die Stirnflanke auch leicht gebogen sein, beispielsweise mit einer leichten nach proximal weisenden Einbuchtung. Die distale Stirnflanke kann eine äußere Querausdehnung aufweisen, die breiter ist als der äußere Querabstand der seitlichen Stützflanken proximal zu ihren, die Taille ausbildenden Einbuchtungen. Insbesondere kann die Querausdehnung der distalen Stirnflanke breiter sein als der äußere Querabstand der Seitenkanten des Elektrodenträgers.

Das erfindungsgemäße Spatelelement kann vollflächig, zum Beispiel in der Form eines flachen Löffels, oder zwischen den seitlichen Stützflanken offen ausgebildet sein. Insbesondere bei einer vollflächigen Ausführung des Spatelelementes ist daran gedacht, dass das Spatelelement als Spritzgussteil hergestellt wird. In einer bevorzugten Ausführungsform ist daran gedacht, dass das Spatelelement aus einer schlaufenförmig gebogenen Stange ausgebildet ist, deren freie Enden fest mit dem Elektrodenträger verbunden sind. Hierbei ist insbesondere daran gedacht, dass das Spatelelement aus einem schlaufenförmig gebogenen Draht hergestellt ist. Unter Stange wird jede allgemeine Form eines länglich ausgedehnten Körpers verstanden. Ein Draht ist eine spezielle Form einer Stange, die aus einem insbesondere biegeflexiblen Metall hergestellt ist. Eine Stange ist nicht auf Metall beschränkt sondern kann aus einem anderen Material wie zum Beispiel einem Kunststoff, einer Keramik oder einem Glas hergestellt sein.

Die freien Enden der gebogenen Stange können proximal zu der Schneidelektrode zusammengeführt sein oder beabstandet voneinander an dem Elektrodenträger befestigt sein. Durch eine Befestigung des Spatelelementes an dem Elektrodenträger proximal zur Schneidelektrode werden sämtliche bei der Manipulation des Gewebes an dem Spatelelement wirkenden Kräfte auf den Elektrodenträger übertragen. Die Schneidelektrode kann dabei unbelastet bleiben.

In einer ersten Variante ist daran gedacht, dass der Elektrodenträger zwei Tragarme aufweist, an denen die Schneidelektrode gelagert ist, wobei insbesondere vorgesehen sein kann, dass die Tragarme rohrförmig ausgebildet sind und dass ein drahtförmiger Körperbereich der Schneidelektrode isolierend in den Tragarmen gehaltert ist, um die mechanische Stabilität der Schneidelektrode zu erhöhen. In dieser Variante können die freien Enden eines aus einer gebogenen Stange oder einem gebogenen Draht ausgebildeten Spatelelementes jeweils an den zwei Tragarmen des Elektrodenträgers befestigt sein. Insbesondere können die freien Enden mit dem Elektrodenträger verklebt oder verschweißt sein.

Bei mit unterschiedlichen Varianten eines Spatelelementes durchgeführten Versuchen hat sich gezeigt, dass ein sich gerade nach distal in einer Ebene erstreckendes Spatelelement besonders gut für die mechanische Abstützung des Gewebes geeignet ist. Die gerade Ausführung der Stützflanken sorgt dafür, dass sich das Gewebe besonders günstig in den seitlichen Taillenbereich der Stützflanken legen kann, sodass bei einer leichten Dreh- und Schubbewegung der Elektrodeneinheit möglichst viel vorgespanntes Gewebe erreicht werden kann. In einer bevorzugten Ausführungsform ist deshalb daran gedacht, dass sich die Stützflanken einschließlich ihrer die Taille ausbildenden Einbuchtungen distal zur Schneidelektrode in einer gemeinsamen Ebene erstrecken.

Bei einem Spatelelement, das aus einem schlaufenförmig gebogenen Draht bzw. aus einer schlaufenförmig gebogenen Stange ausgebildet ist, erstreckt sich die Stange oder der Draht gemäß dieser Ausführungsform in einer gemeinsamen Ebene. Dabei liegen auch die nach innen weisenden Einbuchtungen der seitlichen Stützflanken in dieser gemeinsamen Ebene. Auswölbungen oder Biegungen der Stützflanken quer zu dieser gemeinsamen Ebene sind nicht vorgesehen, da mit solchen quer zu der Spatelebene ausgebildeten Wölbungen das mit dem Spatelelement erfasste Gewebe in unvorteilhafte Richtungen gedrückt werden würde. Oberhalb des Spatelelements soll das erfasste Gewebe möglichst frei überlappen können, um einen unnötigen Kraftaufwand bei der Vorspannung des Gewebes zu vermeiden. Unterhalb des Spatelelementes, auf der Seite der Schneidelektrode, soll das Gewebe durch den Taillenbereich möglichst weit in die Kontaktzone der Schneidelektrode gelangen. Eine nach unten durchhängende Taille würde das Gewebe weiter vom Wurzelbereich der Schneidelektrode entfernen, was den Kontaktbereiches der Elektrode mit dem Gewebe verringert.

Grundsätzlich ist denkbar, dass sich das Spatelelement distal zur Schneidelektrode in einer Ebene erstreckt, die parallel zur Schaftachse des Elektrodenschaftes verläuft. Bei durchgeführten Versuchen hat sich allerdings gezeigt, dass ein schief zur Schaftachse des Elektrodenschaftes ausgerichtetes Spatelelement besonders gut zur Manipulation des zu entfernenden Gewebes geeignet ist. Deshalb ist in einer bevorzugten Ausführungsform vorgesehen, dass sich die Stützflanken distal zur Schneidelektrode in einer gemeinsamen Ebene erstrecken, die schief zur Schaftachse des Elektrodenschaftes verläuft, sodass sich die Stützflanken distal zur Schneidelektrode in einem Winkel geneigt zur Schaftachse des Elektrodenschaftes bzw. in einem Winkel geneigt zur Erstreckungsrichtung des Elektrodenträgers nach distal erstrecken. Dabei ist insbesondere daran gedacht, dass sich die Stützflanken distal zur Schneidelektrode in einem Winkel zwischen 0° und 45° gegenüber der Schaftachse des Elektrodenschaftes bzw. der Erstreckungsrichtung des Elektrodenträgers nach distal erstrecken, und zwar in Erstreckungsrichtung der Schneidelektrode gekippt. Besonders bevorzugt ist ein Winkel zwischen 0° und 20°. Sehr gute Eigenschaften für eine mechanische Abstützung haben sich ergeben bei einer Neigung der Stützflanken in einem Winkelbereich zwischen 5° und 15°. Besonders bevorzugt ist ein Neigungswinkel von 10°.

Die Erstreckungsrichtung der Schneidelektrode verläuft bei herkömmlichen Elektrodeneinheiten in einem Winkel von 90° gegenüber der Schaftachse des Elektrodenschaftes bzw. gegenüber der Erstreckungsrichtung des Elektrodenträgers. Bei der erfindungsgemäßen Elektrodeneinheit hat sich gezeigt, dass eine leicht nach distal weisende Neigung der Schneidelektrode in einem Winkelbereich zwischen 70° und 90°, insbesondere bei einem Winkel von 80° sehr gute Schneidergebnisse in Kombination mit dem erfindungsgemäßen Spatelelement ergeben. Bei einem Neigungswinkel von weniger als 90° erstreckt sich die Schneidelektrode also gegenüber der Schaftachse des Elektrodenschaftes bzw. gegenüber der Erstreckungsrichtung des Elektrodenträgers leicht nach distal gerichtet. Bei einer Schlingenelektrode bezieht sich dieser Neigungswinkel auf die Neigung der Ebene, in der sich die Schneidschlinge erstreckt und bei einer Nadelelektrode bezieht sich der Neigungswinkel auf die Nadelachse in der sich die nadelförmige Schneidelektrode erstreckt.

Die Schneidelektrode kann wie bei herkömmlichen Elektrodeneinheiten zur Prostataentfernung schlingenförmig ausgebildet sein. Dabei ist insbesondere an eine halbkreisförmige Ausbildung der Schneidschlinge gedacht. Grundsätzlich sind auch andere Schlingenformen, wie zum Beispiel eine Oval-Form, eine Dreieck-Form oder eine Rechteck-Form denkbar. Bei dem Einsatz der erfindungsgemäßen Elektrodeneinheit zur Entfernung von krankhaftem Gewebe, wie zum Beispiel einem Tumor aus der Harnblase des Patienten ist bevorzugt daran gedacht, dass die Schneidelektrode nadelförmig ausgebildet ist. Bei einer nadelförmigen Ausbildung der Schneidelektrode steht der in schneidenden Kontakt mit dem Gewebe bringbare Körperteil der Schneidelektrode fingerartig bzw. stiftartig von dem Elektrodenträger ab. Mit einer Nadelelektrode können präzise, furchenartige Schnitte durch das zu entfernende Gewebe gezogen werden, mit denen das Gewebe teilend zerschnitten wird, wohingegen sich mit einer Schneidschlinge raumgreifend längliche Gewebestreifen von der kontaktierten Oberfläche abtragen lassen.

Bevorzugt ist daran gedacht, dass die Schneidelektrode aus einem Draht, insbesondere aus einem gebogenen Draht ausgebildet ist, dessen freie Enden an dem Elektrodenträger befestigt sind. Bei einer nadelförmigen Ausführung der Schneidelektrode kann der Draht an seinem die Eintauchtiefe der Elektrode in das Gewebe bestimmenden Ende um 180° gebogen und eng anliegend zurückgeführt sein, sodass die zwei hin- und zurücklaufenden Drahtschenkel einen fingerförmigen, insbesondere einen geraden nadelförmigen Elektrodenkörper ausbilden.

Zur Vermeidung von elektrischen Überschlägen zwischen der mit einem Hochfrequenzstrom beaufschlagbaren Schneidelektrode und dem Spatelelement kann vorgesehen sein, dass das Spatelelement aus einem elektrisch isolierenden Material, wie zum Beispiel einer Keramik oder einem Kunststoff hergestellt ist. Dabei muss beachtet werden, dass das Spatelelement den bei der Abstützung des Gewebes auftretenden mechanischen Belastungen standhält und eine Überlastung nicht zum Zerbrechen des Spatelelementes führt. Für eine ausreichende Stabilität kann durch entsprechende Materialauswahl gesorgt werden. Bei der Ausbildung besonders geringer Materialstärken des Spatelelementes kann auch ein biegeflexibles Material gewählt werden, damit eine Überlastung zur elastischen Verformung und nicht zum Bruch führt. Gleichzeitig muss das Spatelelement robust genug sein, um das zu schneidende Gewebe kraftvoll abstützen zu können. Außerdem sollte sich das Spatelelement leicht an dem Elektrodenträger befestigen lassen. Vor diesem Hintergrund ist in einer bevorzugten Ausführungsform daher daran gedacht, dass das Spatelelement aus einem Metall hergestellt ist, wobei das Spatelelement zumindest in räumlicher Nähe zur Schneidelektrode mit einer elektrisch isolierenden Materialschicht umhüllt ist. Vorteilhaft ist eine Isolierung des Spatelelementes im Bereich der Schneidelektrode, um einen direkten Eintrag des Hochfrequenzstromes von der Schneidelektrode in das Spatelelement oder einen Kurzschluss bei der Berührung des Spatelelementes mit der Schneidelektrode zu verhindern. Bei der Ausbildung des Spatelelementes aus einem Metalldraht, der die Schneidelektrode zumindest seitlich einfasst, sind vor allem die Stützflankenabschnitte mit den nach innen geformten Ausbuchtungen zur Ausbildung einer Taille im Bereich der Schneidelektrode von besonderem Interesse für eine Isolierung, da diese Bereiche besonders dicht an der Schneidelektrode liegen.

Grundsätzlich könnte eine Isolierung allein der seitlichen Stützflanken im Bereich der Schneidelektrode unter Auslassung der distalen Stirnflanke genügen, um einen unerwünschten Eintrag des Hochfrequenzstromes in das Spatelelement zu vermeiden. In einer Ausführungsform ist jedoch daran gedacht, dass das Spatelelement in seinem gesamten, die Schneidelektrode distal überragenden Bereich, also insbesondere die seitlichen Stützflanken einschließlich der distalen Stirnflanke mit einer elektrisch isolierenden Materialschicht umhüllt ist.

In einem an dem Elektrodenträger anliegenden Bereich kann der Metallkörper des Spatelelementes freiliegen, um eine Verschweißung mit dem ebenfalls typischerweise aus Metall bestehenden Elektrodenträger zu ermöglichen.

Erfindungsgemäß ist auch ein Resektoskop mit einer Elektrodeneinheit nach einem der Ansprüche 1-8. Vorzüge und Details eines erfindungsgemäßen Resektoskops ergeben sich auch aus den Erläuterungen zur erfindungsgemäßen Elektrodeneinheit.

Verwendung findet eine erfindungsgemäße Elektrodeneinheit oder ein Resektoskop mit einer erfindungsgemäßen Elektrodeneinheit in einem Verfahren zur elektrochirurgischen Resektion von Gewebe in einem mit Flüssigkeit gefüllten Hohlraum eines Patienten, insbesondere in der Harnblase eines Patienten.

Bei diesem Verfahren ist daran gedacht, dass die Elektrodeneinheit in den Hohlraum eingeführt wird, die Schneidelektrode mit einem Wechselstrom beaufschlagt wird und das zu resezierende Gewebe oder ein Gewebebereich im Umfeld des zu resezierenden Gewebes mit der mit dem Wechselstrom beaufschlagten Schneidelektrode kontaktiert wird. Bei einer mechanischen Stützung des zu resezierenden Gewebes mit Hilfe des an der Elektrodeneinheit angeordneten Spatelelementes wird eine Spannung im Gewebe erzeugt, die den Schnitt durch das Gewebe erleichtert. Der Schnittbereich wird dabei in die Taille des Spatelelementes geführt und mit der Schneidelektrode zerschnitten. Weitere Vorteile, Merkmale und Einzelheiten der Erfindung ergeben sich auch aus den im Folgenden beschriebenen Ausführungsbeispielen unter Bezugnahme auf schematisierte Zeichnungen. Es zeigen:
- Fig. 1: eine schematische Abbildung einer erfindungsgemäßen Elektrodeneinheit in perspektivischer Ansicht,
- Fig. 2: eine Draufsicht auf den distalen Bereich der Elektrodeneinheit aus Fig. 1 von oben,
- Fig. 3: eine Draufsicht auf den distalen Bereich der Elektrodeneinheit aus Fig. 2 von unten,
- Fig. 4: eine Seitenansicht des Elektrodenträgers mit dem daran befestigten Spatelelement aus den Fig. 2 und 3, und
- Fig. 5: ein Resektoskop mit einer erfindungsgemäßen Elektrodeneinheit.

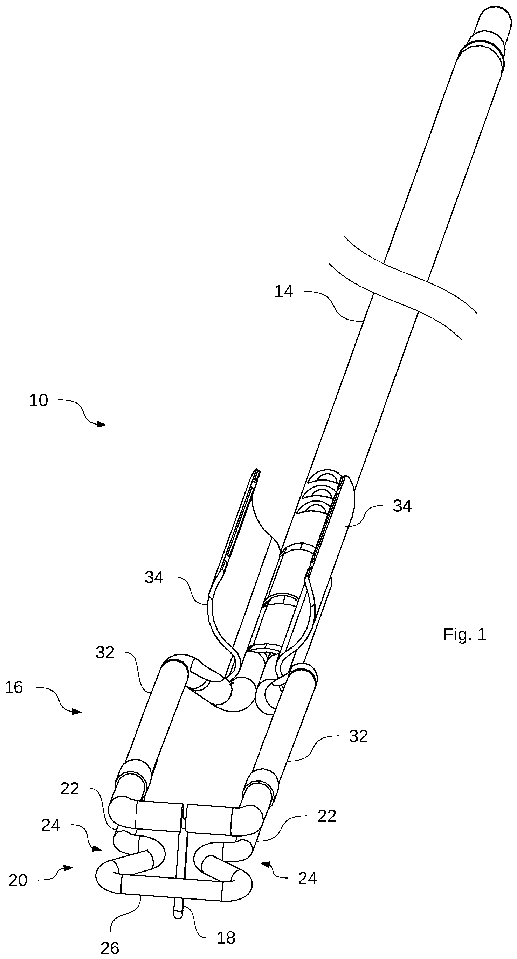

Fig. 1 zeigt eine erfindungsgemäße Elektrodeneinheit 10 für urologische Resektoskope mit einem geraden, langgestreckten stabförmigen Elektrodenschaft 14, der an seinem distalen Ende einen Elektrodenträger 16 mit einer daran befestigten Schneidelektrode 18 aufweist. Der Elektrodenträger 16 ist im vorliegenden Beispiel mit zwei sich gabelförmig angeordneten Tragarmen 32 ausgestattet, die an ihrem distalen Ende die in diesem Fall nadelförmig ausgebildete Schneidelektrode 18 tragen.

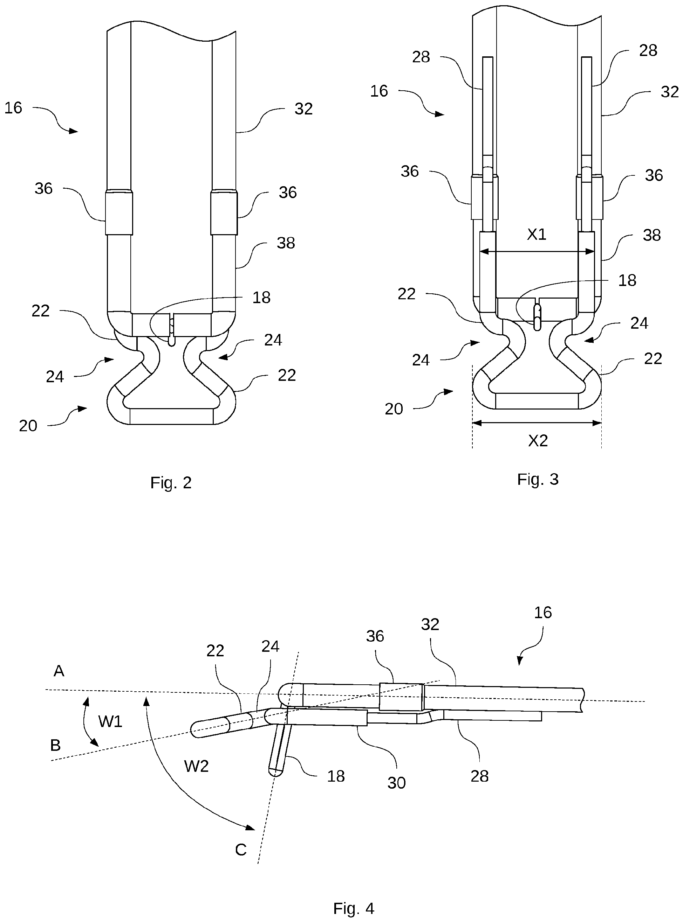

Zur Beaufschlagung der Schneidelektrode 18 mit einem Hochfrequenzstrom ist ein elektrischer Leiter von dem proximalen Ende des Elektrodenschaftes 14 über den Elektrodenträger 16 bis an die Schneidelektrode 18 geführt. Im proximalen Endbereich wird der Elektrodenschaft 14 an eine Kontaktstelle eines Resektoskopes anschließbar, mittels der eine Verbindung mit einem Hochfrequenzgenerator herstellbar ist (nicht dargestellt). Wie sich insbesondere in Zusammenschau mit der Fig. 4 erkennen lässt, trägt der Elektrodenträger 16 neben der Schneidelektrode 18 auch das erfindungsmäße Spatelelement 20, das zwei sich nach distal erstreckende, die Schneidelektrode 18 distal überragende Stützflanken 22 aufweist. Im Bereich der Schneidelektrode 18 weisen die seitlichen Stützflanken 22 zur Ausbildung einer Taille zwei nach innen, aufeinander zugerichtete Einbuchtungen 24 auf. Distal schließt das Spatelelement 20 mit einer quer zur Schaftachse des Elektrodenschaftes 14 ausgerichteten Stirnflanke 26 ab. Die Stirnflanke 26 kann dabei in einer Ebene liegen, die sich parallel zu einer von den Tragarmen 32 des Elektrodenträgers 16 aufgespannten Trägerebene A erstreckt.

Proximal zum Elektrodenträger 16 sind an dem Elektrodenschaft 14 zwei bogenförmig geformte Haltelaschen 34 angeordnet, die zur verschieblichen Lagerung der Elektroneneinheit 10 an einem Schaft oder einer stabförmigen Optikeinheit eines Resektoskopes (nicht dargestellt) vorgesehen sind. Die Haltelaschen 34 bilden eine etwa zangenartige Haltestruktur mit einer in Querrichtung des Elektrodenschaftes 14 freien Öffnung, sodass die Halteelemente 34 unter elastischer Aufweitung auf den Umfang eines rohrförmigen Schaftes aufclipbar sind. Alternativ kann die vorliegend durch die Haltelaschen 34 ausgebildete Haltestruktur auch in einer geschlossenen Rohrform ausgebildet sein, sodass die Elektrodeneinheit 10 für eine verschiebliche Lagerung mit einer als Tragrohr ausgebildeten Haltestruktur auf einen rohrförmigen Schaft oder eine stabförmige Optik eines Resektoskopes aufgeschoben werden kann.

Die Fig. 2 und 3 zeigen eine Detailansicht des Elektrodenträgers 16 mit der daran befestigten Schneidelektrode 18 und dem erfindungsgemäßen Spatelelement 20 aus Fig. 1 in einer Draufsicht von oben (Fig. 2) und von unten (Fig. 3). In Detailansichten der Fig. 2 und 3 ist zu erkennen, dass die vorliegend parallel zueinander verlaufenden Tragarme 32 des Elektrodenträgers 16 jeweils in einen umfangserweiterten Verbindungsabschnitt 36 münden in die die freien Enden der vorzugsweise aus einem Metalldraht ausgebildeten Schneidelektrode 18 aus distaler Richtung eingesteckt sind. Der in die Verbindungsstellen 36 eingesteckte Metalldraht der Schneidelektrode 18 ist bis auf eine zum schneidenden Eingriff mit dem Gewebe vorgesehenen Kontaktzone der Schneidelektrode 18 mit einem elektrisch isolierenden Schutzmantel 38 umhüllt, um einen unerwünschten Eintrag von hochfrequentem Strom in das im Operationsgebiet umliegende Gewebe oder eine Rückleitung des Hochfrequenzstroms in die typischerweise aus Metall bestehende Außenwand des Elektrodenträgers zu verhindern.

Wie die Fig. 2 und 3 verdeutlichen, kann die durch die nach innen geformten Einbuchtungen 24 der seitlichen Stützflanken 22 ausgebildete Taille derart angeordnet sein, dass die engste Stelle zwischen den Stützflanken 22 distal zu der Schneidelektrode 18 platziert ist. Damit kann ein seitlich in die Taille des Spatelelementes 20 geführtes Gewebe leicht nach distal vorgespannt werden, bevor es durch einen Vorschub der Elektrodeneinheit 10 mit der Schneidelektrode 18 kontaktiert wird.

Wie die Figuren 2 und 3 des Weiteren zeigen, verlaufen die in die Stützflanken 22 übergehenden freien Enden 28 des Spatelementes 20 im Bereich des Elektrodenträgers zunächst geradlinig und parallel in distale Richtung, bevor sie etwa auf Höhe der Schneidelektrode 18 jeweils eine Biegung nach innen beginnen, um im weiteren Verlauf die Einbuchtungen 24 zur Taillenbildung auszuformen. An einer Stelle des geringsten äußeren Querabstandes zwischen den Außenkanten der Stützflanken 22 biegen sich die Stützflanken 22 in ihrem weiteren Verlauf nach distal wieder nach außen und erstrecken sich nach schräg distal bis ein äußerer Querabstand X2 der Stützflanken 22 erreicht ist, der größer ist, als der größte Querabstand X1 der Stützflanken 22 proximal zur Schneidelektrode 18.

Die Fig. 4 zeigt eine Seitenansicht des Elektrodenträgers 16 mit dem daran befestigten Spatelelement 20 aus den Fig. 2 und 3. Wie Fig. 4 anschaulich zeigt, erstreckt sich das Spatelelement 20 distal zur Schneidelektrode 18 bevorzugt in einer Ebene B, die in einem Winkel W1 geneigt zu einer von den Tragarmen 32 des Elektrodenträgers 16 aufgespannten Trägerebene A verläuft, wobei die Trägerebene A vorzugsweise parallel oder annähernd parallel zur Schaftachse des Elektrodenschaftes 14 ausgerichtet ist. In dem vorliegenden Beispiel ist die Ebene B, in der sich die seitlichen Stützflanken 22 des Spatelelementes 22 nach distal erstreckt um etwa 10° gegenüber der Trägerebene A geneigt, und zwar in die Erstreckungsrichtung der Schneidelektrode 18 gekippt. Zur Verbesserung der Schneidwirkung bei einem Schnitt mit der Schneidelektrode 18 durch das Gewebe können die Tragarme 32 und damit die Trägerebene A gegenüber der Schaftachse des Elektrodenschaftes 14 geringfügig, zum Beispiel in einem Bereich zwischen 0° bis 3°, vorzugsweise in einem Bereich zwischen 0,5° und 2,5° nach oben ansteigend, also entgegen der Erstreckungsrichtung der Schneidelektrode 18 ausgebildet sein.

Die Schneidelektrode 18 ist vorliegend nadelförmig ausgebildet. Damit steht die Schneidelektrode 18 etwa fingerförmig von dem Elektrodenträger 16 ab. Die Schneidelektrode 18 ist im vorliegenden Beispiel nicht rechtwinklig zur Trägerebene A ausgerichtet sondern verläuft in einem Winkel W2 dazu, der kleiner ist als 90°. Wie vorliegend angedeutet, kann der Winkel W2 zwischen der Trägerebene A und der Erstreckungslinie C der Schneidelektrode 18 bevorzugt 80° betragen.

Wie man aus der Fig. 4 des Weiteren erkennt, sind die freien Enden 28 des Spatelelementes 22 proximal zu den Verbindungsstellen 36 an dem Elektrodenträger 16 befestigt, wobei die freien Enden 28 proximal zu den Verbindungsstellen 36 zunächst eng anliegend an den Trägerarmen 32 geführt sind und in ihrer distalen Erstreckung vor Erreichen der umfangserweiterten Verbindungsstellen 36 des Elektrodenträgers 16 eine Stufe ausbilden, um einen Abstand zu den Verbindungsstellen 36 und des sich distal dazu anschließenden Elektrodenbereiches herzustellen. Dies sorgt für eine mechanische Entlastung der Verbindungsstellen 36 und der in die Verbindungsstellen 36 eingesteckten Drahtenden der Schneidelektrode 18. Die bei einer Manipulation von Gewebe auf das Spatelelement 20 wirkenden mechanischen Kräfte werden somit auf den Elektrodenträger 16 übertragen.

Im Bereich der Schneidelektrode 18 ist das vorzugsweise aus einem gebogenen Draht hergestellte Spatelelement 20 mit einer elektrisch isolierenden Materialschicht 30 ummantelt, um einen direkten oder unmittelbaren Eintrag eines hochfrequenten elektrischen Stroms der Schneidelektrode 18 in den Metallkörper des Spatelelementes 20 und damit in umliegendes Gewebe oder in die Außenwand des Elektrodenträgers 16 zu verhindern. Die metallische Außenwand der Tragarme 32 und ggf. auch die leitend mit den Tragarmen 32 verbundenen freien Enden 28 des Spatelelements 16 dienen vorzugsweise als Rückleiter des beim elektrochirurgischen Schnitt in das Gewebe eingetragenen Hochfrequenzstroms. Ohne eine Isolierung des Spatelelementes 16 im Bereich der Schneidelektrode 18 würde der Hochfrequenzstrom direkt vom oberen Ende der Schneidelektrode 18 in das die Schneidelektrode 18 umgebene Spatelelement 16 fließen. Zusätzlich besteht bei einem aus Metall hergestellten Spatelelementes 16 ohne geeignete Isolierung die Gefahr eines Kurzschlusses zwischen dem Rückleiter und der Schneidelektrode 18 bei einer leitenden Berührung der Schneidelektrode 18 und dem Spatelelement 16 (zum Beispiel durch eine Verbiegung des Spatelelementes 16 bei einer kraftvollen Manipulation von Gewebe).

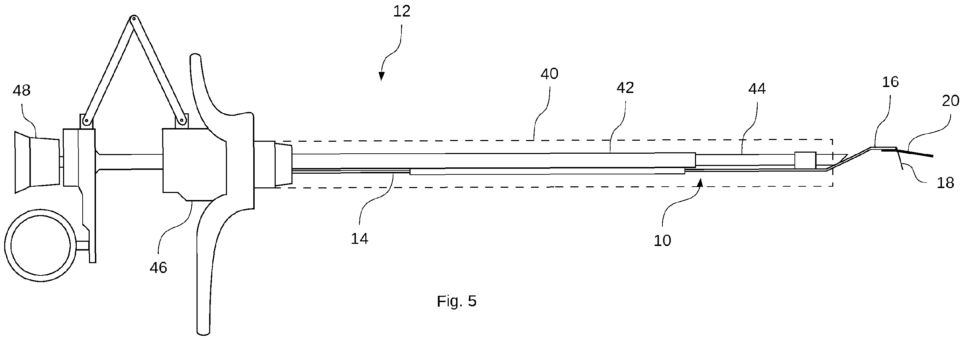

Fig. 5 zeigt ein Resektoskop 12 mit einer in einem Hüllrohr 40 geführten erfindungsgemäßen Elektrodeneinheit 10. Innerhalb des Hüllrohres 40 ist der Elektrodenschaft 14 der Elektrodeneinheit 10 längsverschiebbar an einem als Optikführungsrohr dienenden Innenschaft 42 geführt. Zur Beobachtung des Operationsgebietes dient eine in den Innenschaft 42 eingeschobene stabförmige Optikeinheit 44, die ein Bild aus ihrem distalen Endbereich an eine proximale, vorliegend als Okular ausgebildete Beobachtungsstelle 48 führt.

Zur Bereitstellung weiterer Kanäle zur Flüssigkeitsführung oder zur Führung von Arbeitsinstrumenten kann außerhalb des Hüllrohres 40 und/oder zwischen dem Hüllrohr 40 und dem als Optikführungsrohr dienenden Innenschaft 42 ein weiterer oder mehr als ein weiterer Schaft vorgesehen sein (nicht dargestellt). Insbesondere bei einem Dauerspülresektoskop wird in der Regel ein weiterer Außenschaft auf das Hüllrohr 40 aufgesteckt, der den Innenschaft 42, das Hüllrohr 40 und die dazwischen gelagerte Elektrodeneinheit 10 aufnimmt. Der Zwischenraum zwischen dem Hüllrohr 40 und einem weiteren Außenschaft wird dabei als weiterer Kanal zur Flüssigkeitsführung genutzt.

In seinem proximalen Endbereich ist der Elektrodenschaft 14 mit einem Schlitten 46 gekoppelt, der längsverschieblich gleitend auf dem Innenschaft 42 gelagert ist. Bei einer Betätigung einer mit dem Schlitten 46 einerseits und dem Optikführungsrohr 42 andererseits gekoppelten Griffeinheit kann der Elektrodenträger 16 zusammen mit der Schneidelektrode 18 und dem Spatelelement 20 axial vorschiebend oder zurückziehend bewegt werden.

**Bezugszeichenliste**

| | | | |
|---|---|---|---|
| 10 | Elektrodeneinheit | A | Trägerebene |
| 12 | Resektoskop | B | Erstreckungsebene des Spatelelementes |
| 14 | Elektrodenschaft | | |
| 16 | Elektrodenträger | C | Erstreckungsebene bzw. -linie der Schneidelektrode |
| 18 | Schneidelektrode | | |
| 20 | Spatelelement | | |
| 22 | Stützflanken | W1 | Winkel zwischen Trägerebene und Erstreckungsebene des Spatelelementes |
| 24 | Einbuchtung | | |
| 26 | Stirnflanke | | |
| 28 | freie Enden des Spatelelementes | W2 | Winkel zwischen Trägerebene und Erstreckungsrichtung der Schneidelektrode |
| 30 | isolierende Materialschicht des Spatelementes | | |
| 32 | Tragarme des Elektrodenträgers | | |
| 34 | Haltelaschen | | |
| 36 | Verbindungsstellen | | |
| 38 | Schutzmantel der Elektrode | | |
| 40 | Hüllrohr | | |
| 42 | Optikführungsrohr | | |
| 44 | Optikeinheit | | |
| 46 | Schlitten | | |
| 48 | Okular | | |

## Patentansprüche

1. Elektrodeneinheit (10) für ein medizinisches Resektoskop (12) zur elektrochirurgischen Resektion von Gewebe in einem mit Flüssigkeit gefüllten Hohlraum eines Patienten, aufweisend einen langgestreckten stabförmigen Elektrodenschaft (14), der an seinem distalen Ende einen Elektrodenträger (16) und eine an dem Elektrodenträger (16) befestigte, mit Wechselstrom beaufschlagbare Schneidelektrode (18) aufweist, wobei an dem Elektrodenträger (16) ein zur Abstützung von Gewebe ausgebildetes, die Schneidelektrode (18) distal überragendes Spatelelement (20) angeordnet ist, **dadurch gekennzeichnet, dass** das Spatelement (20) zwei zu beiden Seiten der Schneidelektrode (18) sich nach distal erstreckende, Außenkanten des Spatelelementes (20) ausbildende Stützflanken (22) aufweist, von denen mindestens eine zur Ausformung einer Taille im Bereich der Schneidelektrode (18) eine nach innen geformte Einbuchtung (24) ausbildet.

2. Elektrodeneinheit nach Anspruch 1, **dadurch gekennzeichnet, dass** das Spatelelement (20) an seinem distalen Ende eine gerade, sich quer zur Schaftachse erstreckende Stirnflanke (26) aufweist.

3. Elektrodeneinheit nach einem der Ansprüche 1 oder 2, **dadurch gekennzeichnet, dass** das Spatelelement (20) aus einer schlaufenförmig gebogenen Stange ausgebildet ist, deren freie Enden (28) fest mit dem Elektrodenträger (16) verbunden sind.

4. Elektrodeneinheit nach einem der vorangegangenen Ansprüche, **dadurch gekennzeichnet, dass** sich die Stützflanken (22) distal zur Schneidelektrode (18) in einer gemeinsamen Ebene (B) erstrecken.

5. Elektrodeneinheit nach Anspruch 4, **dadurch gekennzeichnet, dass** die Ebene (B) schief zur Schaftachse des Elektrodenschaftes (14) verläuft, sodass sich die Stützflanken (22) distal zur Schneidelektrode (18) in einem Winkel (W1) geneigt zur Schaftachse des Elektrodenschaftes (14) nach distal erstrecken.

6. Elektrodeneinheit nach einem der vorangegangenen Ansprüche, **dadurch gekennzeichnet, dass** die Schneidelektrode (18) nadelförmig ausgebildet ist.

7. Elektrodeneinheit nach einem der vorangegangenen Ansprüche, **dadurch gekennzeichnet, dass** die Schneidelektrode (18) aus einem Draht, insbesondere aus einem gebogenen Draht ausgebildet ist, dessen freie Enden an dem Elektrodenträger (16) befestigt sind.

8. Elektrodeneinheit nach einem der vorangegangenen Ansprüche, **dadurch gekennzeichnet, dass** das Spatelelement (20) aus einem Metall hergestellt ist, und wobei das Spatelelement (20) zumindest in räumlicher Nähe zur Schneidelektrode (18) mit einer elektrisch isolierenden Materialschicht (30) umhüllt ist.

9. Resektoskop mit einer Elektrodeneinheit nach einem der Ansprüche 1 bis 8.

## Claims

1. Electrode unit (10) for a medical resectoscope (12) for electrosurgical resection of tissue in a liquidfilled cavity of a patient, comprising an elongate rodshaped electrode shaft (14), the distal end of which has an electrode mount (16) and a cutting electrode (18), which is fastened to the electrode mount (16) and to which alternating current is able to be applied, wherein a spatula element (20) which protrudes distally beyond the cutting electrode (18) and is designed to support tissue is arranged on the electrode mount (16), **characterized in that** the spatula element (20) has two support flanks (22) which extend distally on both sides of the cutting electrode (18) and form outer edges of the spatula element (20), at least one of said support flanks forming an inwardly shaped recess (24) for the purposes of forming a waist in the region of the cutting electrode (18) .

2. Electrode unit according to Claim 1, **characterized in that** the distal end of the spatula element (20) has a straight end flank (26) that extends transversely to the shaft axis.

3. Electrode unit according to either one of Claims 1 and 2, **characterized in that** the spatula element (20) is formed from a rod that has been bent in loop-shaped fashion, the free ends (28) of which are securely connected to the electrode mount (16).

4. Electrode unit according to any one of the preceding claims, **characterized in that** the support flanks (22) extend in a common plane (B) distally of the cutting electrode (18).

5. Electrode unit according to Claim 4, **characterized in that** the plane (B) extends obliquely with respect to the shaft axis of the electrode shaft (14) such that the support flanks (22) distally of the cutting electrode (18) extend distally so as to be inclined at an angle (W1) with respect to the shaft axis of the electrode shaft (14).

6. Electrode unit according to any one of the preceding claims, **characterized in that** the cutting electrode (18) has a needle-like form.

7. Electrode unit according to any one of the preceding claims, **characterized in that** the cutting electrode (18) is formed from a wire, in particular a bent wire, the free ends of which are fastened to the electrode mount (16) .

8. Electrode unit according to any one of the preceding claims, **characterized in that** the spatula element (20) is produced from a metal and wherein the spatula element (20) is enveloped by an electrically insulating material layer (30), at least in the spatial vicinity of the cutting electrode (18).

9. Resectoscope having an electrode unit according to any one of Claims 1 to 8.

## Revendications

1. Unité d'électrode (10) pour un résectoscope médical (12) servant à la résection électrochirurgicale de tissus dans une cavité remplie de liquide d'un patient, présentant un corps d'électrode (14) allongé, en forme de tige, qui présente à son extrémité distale un support d'électrode (16) et une électrode de résection (18) fixée au support d'électrode (16) et à laquelle un courant alternatif peut être appliqué, dans laquelle un élément formant spatule (20), réalisé pour soutenir des tissus et dépassant distalement de l'électrode de résection (18), est disposé au niveau du support d'électrode (16),
**caractérisée en ce que** l'élément formant spatule (20) présente deux flancs d'appui (22) s'étendant vers le côté distal des deux côtés de l'électrode de résection (18) et réalisant des bords extérieurs de l'élément formant spatule (20), dont au moins l'un réalise une encoche (24) formée vers l'intérieur afin de former un rétrécissement au niveau de l'électrode de résection (18).

2. Unité d'électrode selon la revendication 1, **caractérisée en ce que** l'élément formant spatule (20) présente à son extrémité distale un flanc frontal droit (26) s'étendant transversalement à l'axe de corps.

3. Unité d'électrode selon l'une quelconque des revendications 1 ou 2, **caractérisée en ce que** l'élément formant spatule (20) est réalisé à partir d'une barre courbée en boucle dont les extrémités libres (28) sont reliées solidement au support d'électrode (16).

4. Unité d'électrode selon l'une quelconque des revendications précédentes, **caractérisée en ce que** les flancs d'appui (22) s'étendent distalement par rapport à l'électrode de résection (18) dans un plan commun (B) .

5. Unité d'électrode selon la revendication 4, **caractérisée en ce que** le plan (B) s'étend en oblique par rapport à l'axe de corps du corps d'électrode (14) de sorte que les flancs d'appui (22) s'étendent distalement par rapport à l'électrode de résection (18) selon un angle (W1) incliné par rapport à l'axe de corps du corps d'électrode (14) vers le côté distal.

6. Unité d'électrode selon l'une quelconque des revendications précédentes, **caractérisée en ce que** l'électrode de résection (18) est réalisée en forme d'aiguille.

7. Unité d'électrode selon l'une quelconque des revendications précédentes, **caractérisée en ce que** l'électrode de résection (18) est réalisée en fil métallique, en particulier en fil métallique plié dont les extrémités libres sont fixées au support d'électrode (16).

8. Unité d'électrode selon l'une quelconque des revendications précédentes, **caractérisée en ce que** l'élément formant spatule (20) est fabriqué en métal, et dans lequel l'élément formant spatule (20) est enveloppé d'une couche de matière électriquement isolante (30), au moins à proximité physique de l'électrode de résection (18).

9. Résectoscope comprenant une unité d'électrode selon l'une quelconque des revendications 1 à 8.
